# EUROPEAN PATENT SPECIFICATION

(11) **EP 4 341 240 B1**
(45) Date of publication and mention of the grant of the patent: **30.09.2026**
(21) Application number: 21940146.0
(22) Date of filing: 19.05.2021
(51) Int. Cl.: C07C 209/84, C07C 213/10

(54) **PROCESSES FOR PURIFYING ORGANIC AMINES**
VERFAHREN ZUR REINIGUNG ORGANISCHER AMINE
PROCÉDÉS DE PURIFICATION D'AMINES ORGANIQUES

(43) Date of publication of application: 27.03.2024
(73) Proprietor: Dow Global Technologies LLC, Midland, MI 48674 (US); DOW CHEMICAL KOREA LIMITED, Seoul 06170 (KR)
(72) Inventor: JIANG, Qi, Shanghai 201203 (CN); OHBA, Kaoru, Tokyo 140 (JP); LIU, Yujun, Lake Jackson, Texas 77566 (US); KIM, Jongcheol, Seoul, 06170 (KR); CHEN, Xue, Lake Jackson, Texas 77566 (US); MU, Li, Shanghai 201203 (CN)
(74) Representative: Boult Wade Tennant LLP
(86) International application number: PCT/CN2021/094704
(87) International publication number: WO 2022/241690

(56) References cited:
- CN-A- 1 669 616
- CN-A- 103 102 273
- CN-A- 103 102 273
- CN-A- 103 412 065
- CN-A- 103 979 497
- CN-A- 104 610 061
- CN-A- 109 160 876
- CN-A- 109 251 143
- GB-A- 774 789
- JP-A- H06 173 054
- US-A1- 2007 043 217

## Description

### FIELD

The present invention relates to processes for purifying organic amines by removing metal contaminants and other impurities.

### INTRODUCTION

Organic amines are good ligands for metal ions and thus, metal impurities are a common issue when producing organic amines. For electronic applications, metal and other contaminants can be major root causes of electronic device failure. The processing chemicals must contain extremely low concentration of impurities such as metals. From prior art and industrial experience, single or multiple purification processes are essential for purifying chemicals to achieve electronic grade standards. Configuration of relevant facilities is complicated and not use-friendly. This invention claims a purification process which is easy to implement and enables manufacturing pure organic amines.

It would be desirable to have a purification process which is easy to implement and facilitates the manufacture of highly pure organic amines. US 2007/0043217 A1 discloses a method for the distillative separation of mixtures containing ethyleneamines.

### SUMMARY

The present invention is directed to processes for purifying organic amines. In various embodiments, the present invention can purify the organic amines to very low levels of metallic ions and other contaminants. In some embodiments, the present invention advantageously provides processes for purification of organic amines that are easier to implement than prior approaches.

In one embodiment, a process for purifying organic amines comprises (a) providing an organic amine to a first vessel, the organic amine having a normal boiling point at one bar; (b) filling the first vessel with inert gas; (c) heating the organic amine in the first vessel to a sub-boiling temperature, wherein the sub-boiling temperature is at least 15° C less than the normal boiling point; (d) cooling the vapor from the first vessel in a second vessel to provide a liquid; and (e) contacting the organic amine with a resin polymer matrix, wherein the resin polymer matrix is embedded with an amino compound selected from the group consisting of iminodiacetic acid, aminomethylphosphonic acid or a combination thereof.

Various embodiments of the present invention are described in more detail in the following Detailed Description.

### BRIEF DESCRIPTION OF FIGURE

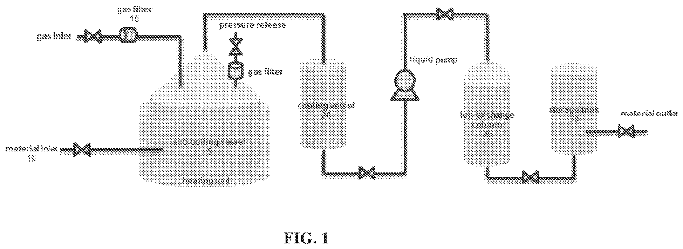

FIG. 1 is a flow diagram illustrating a process for purifying organic amines according to one embodiment of the present invention.

### DETAILED DESCRIPTION

As used throughout this specification, the abbreviations given below have the following meanings, unless the context clearly indicates otherwise: BV/hr = bed volume/hour(s), µm = micron(s), nm = nanometer(s), g = gram(s); mg = milligram(s); L = liter(s); mL = milliliter(s); ppm = parts per million; ppb = parts per billion; ppt = parts per trillion; m = meter(s); mm = millimeter(s); cm = centimeter(s); min = minute(s); s = second(s); hr = hour(s); °C = degree(s) Celsius; % = percent, vol % = volume percent; and wt % = weight percent.

In general, the present invention relates to processes for purifying organic amines. The organic amines which can be purified by use of these processes include, but are not limited to highly concentrated (with less than 1% by weight water, preferably less than 0.1%) N-methylethanolamine or the similar chemical structures, such as monoethanolamine, diethanolamine, triethanolamine, isopropanolamine, diisopropanolamine, triisopropanolamin, N-methyldiethanolamine, aminoethyleneethanolamine, etc. These close to pure amines may also be mixed together. In some embodiments, the viscosity of the organic amines to be purified ranges from 10 cP to 100 cP (1 cP = 1mPa.s) (as measured by ASTM D7042), with a pH value of 0.1 mol/L aqueous solution ranging from 10-13 (as measured by ASTM E70). Among the properties important in characterizing the organic amines for use in the inventive processes is the normal boiling point. As used herein, the "normal boiling point" is the boiling point of the organic amine measured at one bar.

In one aspect, a process for purifying organic amines (as described herein) comprises (a) providing an organic amine to a first vessel, the organic amine having a normal boiling point at one bar; (b) filling the first vessel with inert gas, wherein the inert gas is nitrogen or argon; (c) heating the organic amine in the first vessel to a sub-boiling temperature, wherein the sub-boiling temperature is at least 15° C less than the normal boiling point; (d) cooling the vapor from the first vessel in a second vessel to provide a liquid; (e) contacting the organic amine with a resin polymer matrix, wherein the resin polymer matrix is embedded with an amino compound selected from the group consisting of iminodiacetic acid, aminomethylphosphonic acid or a combination thereof, wherein a minimum sub-boiling temperature is 160° C less than the normal boiling point if the normal boiling point of the organic amine is at least 200° C, wherein a minimum sub-boiling temperature is 120° C less than the normal boiling point if the normal boiling point of the organic amine is between 150° C and 200° C, and wherein a minimum sub-boiling temperature is greater than 25° C if the normal boiling point of the organic amine is less than 150° C. In some embodiments, steps (c) and (d) are performed before step (e), with the organic amine in step (e) being the liquid from step (d). In other words, in such embodiments, the sub-boiling separation is performed before contact with the resin polymer matrix. In other embodiments, step (e) is performed before steps (a)-(d), wherein the organic amine is provided to the first vessel in step (a) after contacting the resin polymer matrix. In other words, in such embodiments, the contact with the resin polymer matrix occurs prior to the sub-boiling separation.

In some embodiments, after the process steps are completed, the concentration of Na, K, Ca, Al, Fe, Ni, Zn, Cu, Cr, and Sn in the organic amine are each 10 ppb or less. In some embodiments, prior to entering a process of the present invention the total metal content (Na, K, Ca, Al, Fe, Ni, Zn, Cu, Cr, and Sn) in the organic amine is up to 5 ppm. In some embodiments, after the process steps are completed, the total metal content (Na, K, Ca, Al, Fe, Ni, Zn, Cu, Cr, and Sn) in the organic amine is 20 ppb or less.

In some embodiments, the water content and the oxygen content in the first vessel are each less than 20 ppm.

In some embodiments, the resin polymer matrix comprises polyacrylate or polystyrene-divinylbenzene. The pore size of the resin polymer matrix, in some embodiments, ranges from 1 - 2,000 nm as determined by specific surface area of solids by gas adsorption. In some embodiments, the resin polymer matrix is introduced to the organic amine containing liquid as resin beads, and the particle diameter of said beads ranging in size from 100 - 2000 µm.

Processes of the present invention include a sub-boiling step. The sub-boiling step involves heating the organic amine to a temperature that is at least 15° C lower than the normal boiling point of the organic amine. The minimum temperature to use for the organic amine in the sub-boiling step will depend on the normal boiling point, and melting point (e.g., the sub-boiling temperature would clearly need to be above the temperature at which the liquid organic amine may crystallize) of the organic amine. In some embodiments, if the normal boiling point of the organic amine is at least 200° C, then the minimum sub-boiling temperature is 160° C less than the normal boiling point. In some embodiments, if the normal boiling point of the organic amine is between 150° C and 200° C, then the minimum sub-boiling temperature is 120° C less than the normal boiling point. In some embodiments, if the normal boiling point of the organic amine is less than 150° C, then the minimum sub-boiling temperature is greater than 25° C. In some embodiments, if the normal boiling point of the organic amine is at least 200° C, then the minimum sub-boiling temperature is 160° C less than the normal boiling point; if the normal boiling point of the organic amine is between 150° C and 200° C, then the minimum sub-boiling temperature is 120° C less than the normal boiling point; and if the normal boiling point of the organic amine is less than 150° C, then the minimum sub-boiling temperature is greater than 25° C.

Turning to one embodiment of the sub-boing step, the organic amine is provided to a first vessel. The first vessel is then filled with an inert gas such as nitrogen or argon. The purity of the inert gas is at least 99.999%. As the inert gas flows into the first vessel, it should pass through a gas filter to remove particles and dust in order to maintain the purity of the gas. In addition, the water content and oxygen content are controlled to less than 20 ppm using techniques known to those having ordinary skill in the art based on the teachings herein. The contents of the first vessel are then heated to a temperature that does not exceed a sub-boiling temperature that is 15° C less than the normal boiling point of the organic amine. The heating of the organic amine in the first vessel generates a vapor. The vapor flows out of the first vessel through a pipe or other conduit into a second vessel. In the second vessel, the vapor is allowed to cool naturally and condense into a liquid. For the organic amines contemplated herein, in some embodiments, the temperature of the liquid in the second vessel should be kept no higher than 20° C. Thus, the sub-boiling procedure, in some embodiments, comprises (a) providing the organic amine to a first vessel; (b) filling the first vessel with inert gas; (c) heating the organic amine in the first vessel to a sub-boiling temperature, wherein the sub-boiling temperature is at least 15° C less than the normal boiling point of the organic amine; and (d) cooling the vapor from the first vessel in a second vessel to provide a liquid.

If the organic amine had not been contacted with a resin polymer matrix prior to sub-boiling, the purified organic amine may be collected for use. If the organic amine has not passed through the resin polymer matrix, the organic amine from the second vessel in the sub-boiling step may proceed to the resin polymer matrix procedure as described further herein.

Contacting the organic amine with a resin polymer matrix entails the use of an ion exchange resin featuring iminodiacetic acid or aminomethylphosphonic acid (or both). Iminodiacetic acid, HN(CH₂CO₂H)₂, often abbreviated to IDA, is a dicarboxylic acid amine. The iminodiacetate anion can act as a tridentate ligand to form a complex with metal ions. Aminomethylphosphonic acid, CH₆NO₃P, abbreviated to (AMPA) is a weak organic acid with a phosphonic acid group which is capable of binding different metal ions mainly through oxygen atoms of the phosphonic acid group.

The ion exchange resin, in a preferred embodiment, may be described as a polymer matrix comprised of polyacrylate or polystyrene-divinylbenzene (or a mixture of the two). The IDA and/or AMPA is embedded within, throughout, and/or upon this polymer matrix. The IDA and/or AMPA may be introduced during formation of the polymer resin and this resin may be formed into beads resulting in the AMPA or IDA embedded inside the resin beads and on the surface. The AMPA or IDA may also be applied at a later step after the resin matrix is formed, resulting in a surface coating only. In a preferred embodiment, the concentration of AMPA or IDA in a resin ranges from 20 wt.% to 70 wt.% and more preferably from 40 wt.% to 60 wt.%. Generally, the higher concentration of AMPA or IDA utilized result in higher metal removal rate, however if the concentration is too high, the polymer matrix may become unstable.

The pore size of the polymer matrix may vary, with one embodiment having a preferred range from 1 - 2000 nanometers. This pore size is determined via ISO 9277:2010, the determination of the specific surface area of solids by gas adsorption (the BET method). The IDA/AMPA resin polymer matrix may be formed into beads, with the distribution of particle diameter ranging from 100 - 2,000 microns. IDA and/or AMPA embedded resins can be mixed with each other at a ratio 100:0 to 0:100. Consistent bead size may be obtained by use of a few meshes with different pore sizes to filter the uniform size of resin bead step by step

Additionally, anion ion exchange resins can also be mixed with the IDA and/or AMPA embedded chelation ion exchange resins. Two such anion ion exchange resins are Amberlite IRA98 (methanaminium N,N,N-trimethyl hydroxide) and Amberjet 9000OH (quaternary ammonium). The anion ion exchange resin is introduced to release hydroxyl anion (OH⁻). This step with anion resin is optional and does not reduce metal removal. Some metals in organic amines exist in a complex form and require a chelating resin with stronger complexing strength. The additional anion resin may not directly capture the complex metals, but they may act as de-complexing agent. The mechanism for this de-complexing, known in the art, releases OH⁻ to form metal hydroxide which can be easier to capture by chelating resins.

When purifying organic amines, the presently disclosed process may feature the use of at least one ion exchange column filled with iminodiacetic acid containing resin or aminomethylphosphonic embedded resin beads. This column may be fluidly connected in line or parallel to another ion exchange column filled with the other material (that is, an aminomethylphosphonic embedded resin or an iminodiacetic acid containing resin, respectively. The organic amine containing liquid is passed through these columns, in one embodiment, at a flow rate of 1 to 30 bed volume (BV) per hour. When used together in series, either of these columns can be placed upstream of the other. Additionally, other column(s) may be loaded with anion ion exchange resin(s) and connected upstream or downstream of the IDA and/or AMPA ion exchange column(s), passing the organic amine containing liquid through the series of columns and producing extremely pure organic amines. As used herein, "BV" means bed volume, and refers to an amount of liquid contacted with the same amount of a hydrated wet mixed bed of ion exchange resin. For example, if 120 mL of a hydrated wet mixed bed of ion exchange resin is used, 1 BV means 120 mL of organic amine is contacted with the mixed bed of ion exchange resin. "BV/hr" is calculated by flow rate (mL/hr) divided by bed volume (mL).

In another embodiment, simple mixing of the ion exchange resin(s) with the organic amine liquid may also be utilized to purify the organic amines. Once mixed, the resin(s) are allowed to react with the organic amines and remove metal from them. Then, liquid is then filtered to separate the purified organic amines from the other components in the liquid.

In general, the temperature of the process during the step of contacting the organic amine with the resin polymer matrix can include, for example, from 0 °C to 100 °C in one embodiment, from 10 °C to 60 °C in another embodiment, and from 20 °C to 40 °C in still another embodiment. If the temperature is above 100 °C, the resin will be damaged; and if the temperature is below the freezing point of the organic amines, the organic amines to be treated may not flow.

FIG. 1 illustrates a process for purifying organic amines according to one embodiment of the present invention. In the embodiment shown in FIG. 1, the process includes a sub-boiling step followed by an ion exchange step (contacting with resin polymer matrix). As noted above, in other embodiments, the ion exchange step (contacting with resin polymer matrix) can be first, followed by the sub-boiling step. Turning to operation of the embodiment shown in FIG. 1, the organic amine(s) are loaded into the sub-boiling vessel 5 at material inlet 10. The sub-boiling vessel 5 is then filled with inert gas, such as nitrogen and/or argon. The purity of the inert gas is at least 99.999% in some embodiments. To remove particles and dust and keep the inert gas clean, the inert gas passes through gas filter 15. The water content and oxygen content inside the vessel is controlled so that each are less than 20 ppm. The organic amine in the sub-boiling vessel 5 is heated to a sub-boiling temperature of the organic amine, wherein the sub-boiling temperature is at least 15° C less than the normal boiling point of the organic amine. In some embodiments, if the normal boiling point of the organic amine is at least 200° C, then the minimum sub-boiling temperature is 160° C less than the normal boiling point; if the normal boiling point of the organic amine is between 150° C and 200° C, then the minimum sub-boiling temperature is 120° C less than the normal boiling point; and if the normal boiling point of the organic amine is less than 150° C, then the minimum sub-boiling temperature is greater than 25° C. The pressure in the sub-boiling vessel 5 can be under vacuum or at ambient pressure, in some embodiments. In some embodiments, the pressure can be higher than ambient pressure, for example, due to the gas inlet pressure. The sub-boiling vessel includes a pressure release valve to prevent the accumulation of pressure (e.g., for safety) with a gas filter to prevent particles from entering the air when pressure (gas) is released from the sub-boiling vessel 5. Heating of the organic amine in the sub-boiling vessel generates vapor which then flows into a cooling vessel 20. In the cooling vessel 20, the vapor condenses into a liquid after cooling naturally. In some embodiments, the temperature in the cooling vessel is kept lower than 60° C. From the cooling vessel 20, the organic amine can be pumped through an ion exchange column 25 for further reduction of metal content. The ion exchange column is loaded with a resin polymer matrix embedded with an amino compound selected from the group consisting of iminodiacetic acid, aminomethylphosphonic acid or a combination thereof as described above. The flow rate of the organic amine through the ion exchange column, in some embodiments, is no more than 50 bed volumes per hour. Upon exiting the ion exchange column 25, the purified organic amine can be stored in a storage tank 30.

In some embodiments, the whole system including the sub-boiling vessel 5, the cooling vessel 20, the ion exchange column 25, the storage tank 30, and all connecting pipelines are made of SAE 316L grade stainless steel with electroplating, or made of ultrapure perfluoroalkoxy alkane (PFA) or polytetrafluoroethylene (PTFE) polymers. Optionally, in some embodiments, such construction materials can be heatproof materials which can withstand temperatures over 250° C, with inner surfaces coated with ultrapure PFA or PTFE having a coating thickness of at least 2 mm.

In one general embodiment, the targeting metal level of the organic amine, after the above-described process (sub-boiling and ion exchange), is less than 20 ppb (part per billion) when the feed solvent contains a typical metal level. The obtained organic amine includes quite low levels of metallic and non-metallic ionic contaminants. The metallic contaminants can include, for example, Na, K, Ca, Al, Fe, Ni, Zn, Cu, Cr, and Sn. The concentration of each of these metallic contaminants can be 10 ppb or less in various embodiments, and 5 ppb or less in other embodiments. Therefore, the organic amines obtained using the process of the present invention can be useful in applications which requires an ultrapure product, such as for the manufacture of pharmaceuticals and electronic materials, and especially for use, for example, in semiconductor fabrication processes. High removal rate of metals is necessary to achieve ultrapure product. In some embodiments, a process of the present invention advantageously provides more than 80% of metal removal efficiency of the sum of the metals listed above from the organic amine ether fed to the process. In some embodiments, a process of the present invention advantageously provides more than 90% of metal removal efficiency of the sum of the metals listed above from the organic amine fed to the process. In some embodiments, a process of the present invention advantageously provides more than 95% of metal removal efficiency of the sum of the metals listed above from the organic amine fed to the process.

It is also desired that the purity change of the organic amine after undergoing a process according to some embodiments of the present invention is as low as possible as measured by conventional methods such as by GC-FID. For example, in some embodiments, the purity change of the organic amines is zero percent (%) or at a level that is lower than the detection limit of a detection instrument (for example, close to zero % such as 0.0001% depending on the selection of the GC detector, selection of the column, and the selection of other measurement conditions). In other embodiments, the purity change of the organic amines after ion-exchange treatment is, for example, less than 0.05 % in one embodiment; and less than 0.01 % in another embodiment.

### EXAMPLES

Some embodiments of the present invention are described in detail in the following Examples. However, the following examples are presented to further illustrate the present invention in detail but are not to be construed as limiting the scope of the claims. Unless otherwise indicated, all parts and percentages are by weight.

Various terms and designations used in the Inventive Examples ("IE") and the Comparative Examples ("CE") are explained as follows:
"DVB" stands for divinyl benzene.

"BV/hr" stands for bed volume/hour(s).

Various raw materials or ingredients used in the Examples are explained as follows:
N-methylethanolamine (NMEA), commercially available from The Dow Chemical Company.

N-methyldiethanolamine (MDEA), commercially available from The Dow Chemical Company.

PUROMET MTS9300H is an iminodiacetic acid chelation resin commercially available from Purolite. PUROMET MTS9500H is an aminomethylphosphonic acid chelation resin commercially available from Purolite. Additional details about these ion exchange resins are provided in Table 1:

**Table 1**

| | **PUROMET MTS9300H** | **PUROMET MTS9500H** |
|---|---|---|
| **Type** | Weak Acid Cation | Weak Base Anion |
| **Form** | Macro-reticular | Macro-reticular |
| **Matrix** | Styrene-DVB | Styrene-DVB |
| **Functional group** | Iminodiacetic acid | Aminomethylphosphonic acid |
| **Ionic** | N(CH₂COO⁻H⁺)₂ | NH⁺CH₂P(OO⁻H⁺)OH⁻ |
| **Moisture retention capacity** | 52-60 % | 60-68 % |
| **Particle size** | 425-1000 µm | 300-1200 µm |

For the Inventive Examples, a sub-boiling step is performed first. The whole system including the sub-boiling vessel, the cooling vessel, the ion-exchange column, bottles, and connecting pipelines are all made of perflouroalkoxy alkane (PFA) materials.

The vessel for sub-boiling has a volume of four liters. A heating bowl is placed below the sub-boiling vessel to heat the material in vessel. The sub-boiling vessel with heating bowl is placed in a glove box filled with ultrapure argon (assay 99.999%) to control oxygen and moisture to < 5 ppm. Particle control is at 100 class clean room level. The pressure is ~1.5 bar.

### Inventive Examples 1-3 (IE1-IE3) and Comparative Examples 1-3 (CE1-CE3)

NMEA is evaluated in Inventive Examples 1-3 and Comparative Examples 1-3. The normal boiling point of NMEA is 156° C, and it has a purity of over 99.0%. Comparative Example 1 is the NMEA without being subjected to sub-boiling or ion exchange.

For the Inventive Examples, three liters of NMEA are added to the sub-boiling vessel. The NMEA is heated to a sub-boiling temperature of 70-90° C. As a result of the heating, vapor is formed in the sub-boiling vessel and flows out the top of the sub-boiling vessel to a four-liter cooling vessel maintained at a temperature of 20° C or less. In the cooling vessel, the vapor condenses into a liquid. For Inventive Examples 1-3, samples from the cooling vessel are collected and tested for purity, metal content, and water content. The water content is measured in accordance with ASTM E203 using Karl Fischer titration. The concentrations of metals in the solvent samples are analyzed by conventional equipment such as an ICP-MS (Inductively Coupled Plasma-mass spectrometry) instrument available from Agilent Technology; and the analytical results are described in the tables which follows herein below. Original metal level (concentration) and metal element ratio are varied by feed solvent lot. Purity is measured by GC (gas chromatography). The purity is calculated dividing the peak area of the major component (100% minus the sum of all impurities including water) divided by the sum of all peak areas.

Inventive Example 3 is passed through an ion exchange column as follows. The ion exchange column has a volume of 100 milliliters. 10 milliliters of the ion exchange resins identified in Table 1 are loaded into the ion exchange column, using 50% of each resin. The flow rate of the sub-boiled organic amine is 6 bed volumes per hour for Inventive Example 3. Comparative Example 2 is NMEA, but a different lot of NMEA from Comparative Example 1. Comparative Example 3 is Comparative Example 2 passed through the ion exchange column like Inventive Example 3, but Comparative Example 3 did not undergo a sub-boiling step. After passing through the ion exchange column, the organic amines are collected in a sample bottle, and the purity, the water content, and the metal content are measured as described above.

The purity, water content, and metal content measurements are shown in Table 2:

**Table 2**

| **Tested Property** | **CE1** | **IE1** | **IE2** | **CE2** | **CE3** | **IE3** |
|---|---|---|---|---|---|---|
| Purity (%) | 99.13 | 99.19 | 99.38 | 98.95 | 98.96 | 99.20 |
| Water content (ppm) | 1103 | 856 | 794 | 1385 | 1517 | 890 |
| Sodium (ppb) | 3.4 | 0.56 | 0.92 | 9.7 | 0.67 | 0.00 |
| Sodium removal rate (%) | | 83.5% | 72.9% | | 92.9% | 100% |
| Calcium (ppb) | 4.4 | 1.18 | 4.31 | 56.4 | 4.6 | ND |
| Calcium removal rate (%) | | 73.2% | 2.0% | | 91.8% | 100% |
| Manganese (ppb) | 2.6 | 0.04 | 0.05 | 0.46 | 0.24 | 0.01 |
| Manganese removal rate (%) | | 98.5% | 80.8% | | 47.8% | 99.7% |
| Iron (ppb) | 186.2 | 0.43 | 1.03 | 15.37 | 3.2 | 0.98 |
| Iron removal rate (%) | | 99.8% | 99.4% | | 89.2% | 99.5% |
| Nickle (ppb) | 7.0 | 3.31 | 6.63 | 0.38 | 0.28 | 0.03 |
| Nickle removal rate (%) | | 52.7% | 5.3% | | 21.6% | 99.6% |
| Copper | 4.8 | 0.13 | 0.10 | 7.16 | 0.81 | 0.13 |
| Copper removal rate (%) | | 97.3% | 97.9% | | 88.9% | 97.3% |
| Zinc (ppb) | 12.4 | 1.04 | 1.33 | 14.07 | 3.6 | ND |
| Zinc removal rate (%) | | 91.6% | 89.3% | | 80.9% | 100% |
| Total metal (ppb): Li, Na, Mg, Al, K, Ca, Cr, Mn, Fe, Co, Ni, Cu, Zn | 223.5 | 7.99 | 15.85 | 136.1 | 14.45 | 2.21 |
| Total metal removal rate (%) | | 96.4% | 92.9% | | 88.6% | 99.0% |

As shown in Table 2, the Inventive Examples contain much less metal than the Comparative Examples without the sub-boiling step. The removal rates of most metals in the Inventive Examples are over 80%. However, the Ca and Ni removal rates of Inventive Examples 1-2 are lower than the removal rates of the other metals. However, the addition of the ion exchange step in Inventive Example 3 increased the Ca and Ni removal rates significantly.

### Inventive Example 4 (IE4) and Comparative Example 4 (CE4)

MDEA is evaluated in Inventive Example 4 and Comparative Example 4. The normal boiling point of DMEA is 243° C, and it has a purity of over 99.0%. Comparative Example 1 is the DMEA without being subjected to sub-boiling or ion exchange.

For Inventive Example 4, three liters of DMEA are added to the sub-boiling vessel. The DMEA is heated to a sub-boiling temperature of 90° C. As a result of the heating, vapor is formed in the sub-boiling vessel and flows out the top of the sub-boiling vessel to a four-liter cooling vessel maintained at a temperature of 20° C or less. In the cooling vessel, the vapor condenses into a liquid. For Inventive Example 4, a sample from the cooling vessel is collected and tested for metal content using the technique described above.

The metal content measurements are shown in Table 3:

**Table 3**

| Tested Property | CE4 | IE4 |
|---|---|---|
| Sodium (ppb) | 17.7 | 0.3 |
| Sodium removal rate (%) | | 98.3% |
| Calcium (ppb) | 14.4 | 5.9 |
| Calcium removal rate (%) | | 60.0% |
| Manganese (ppb) | 1.6 | 0.0 |
| Manganese removal rate (%) | | 100% |
| Iron (ppb) | 33.3 | 0.4 |
| Iron removal rate (%) | | 98.8% |
| Nickle (ppb) | 0.1 | 0.1 |
| Nickle removal rate (%) | | N/A |
| Copper | 1.1 | 0.1 |
| Copper removal rate (%) | | 90.9% |
| Zinc (ppb) | 552.0 | 0.8 |
| Zinc removal rate (%) | | 99.9% |
| Total metal (ppb): Li, Na, Mg, Al, K, Ca, Cr, Mn, Fe, Co, Ni, Cu, Zn | 626.4 | 12.5 |
| Total metal removal rate (%) | | 96.4% |

As shown in Table 3, the sub-boiling step removed over 96% of the metal content from the DMEA.

## Claims

1. A process for purifying organic amines, the process comprising:
(a) providing an organic amine to a first vessel, the organic amine having a normal boiling point at one bar;
(b) filling the first vessel with inert gas, wherein the inert gas is nitrogen or argon;
(c) heating the organic amine in the first vessel to a sub-boiling temperature, wherein the sub-boiling temperature is at least 15° C less than the normal boiling point;
(d) cooling the vapor from the first vessel in a second vessel to provide a liquid; and
(e) contacting the organic amine with a resin polymer matrix, wherein the resin polymer matrix is embedded with an amino compound selected from the group consisting of iminodiacetic acid, aminomethylphosphonic acid or a combination thereof,
wherein a minimum sub-boiling temperature is 160° C less than the normal boiling point if the normal boiling point of the organic amine is at least 200° C, wherein a minimum sub-boiling temperature is 120° C less than the normal boiling point if the normal boiling point of the organic amine is between 150° C and 200° C, and wherein a minimum sub-boiling temperature is greater than 25° C if the normal boiling point of the organic amine is less than 150° C.

2. The process of claim 1, wherein steps (c) and (d) are performed before step (e), and wherein the organic amine in step (e) is in the liquid from step (d).

3. The process of claim 1, wherein step (e) is performed before steps (a)-(d), and wherein the organic amine is provided to the first vessel in step (a) after contacting the resin polymer matrix.

4. The process of any of the preceding claims, wherein after the process steps are completed, the concentration of Na, K, Ca, Al, Fe, Ni, Zn, Cu, Cr, and Sn in the organic amine are each 10 ppb or less.

5. The process of any of the preceding claims, wherein water content and oxygen content in the first vessel are each less than 20 ppm.

6. The process of any of the preceding claims, wherein the resin polymer matrix comprises polyacrylate or polystyrene-divinylbenzene.

7. The process of any of the preceding claims, wherein the pore size of the resin polymer matrix ranges from 1 - 2,000 nm as determined by specific surface area of solids by gas adsorption.

8. The process of any of the preceding claims, wherein the resin polymer matrix is introduced to the organic amine containing liquid as resin beads, the particle diameter of said beads ranging in size from 100 - 2000 µm.

9. The process of any of the preceding claims, wherein the organic amine includes highly concentrated monoethanolamine, diethanolamine, triethanolamine, isopropanolamine, diisopropanolamine, triisopropanolamin, dimethylethanolamine, N-methyldiethanolamine, or aminoethyleneethanolamine.

## Patentansprüche

1. Verfahren zum Aufreinigen organischer Amine, das Verfahren umfassend:
(a) Bereitstellen eines organischen Amins an ein erstes Gefäß, wobei das organische Amin einen Normalsiedepunkt bei einem Bar aufweist;
(b) Füllen des ersten Gefäßes mit Inertgas, wobei das Inertgas Stickstoff oder Argon ist;
(c) Erhitzen des organischen Amins in dem ersten Gefäß auf eine Subsiedetemperatur, wobei die Subsiedetemperatur mindestens 15 °C weniger als der Normalsiedepunkt ist;
(d) Kühlen des Dampfes aus dem ersten Gefäß in einem zweiten Gefäß, um eine Flüssigkeit bereitzustellen; und
(e) Inberührungbringen des organischen Amins mit einer Harzpolymermatrix, wobei in die Harzpolymermatrix eine Aminoverbindung eingebettet ist, ausgewählt aus der Gruppe, bestehend aus Iminodiessigsäure, Aminomethylphosphonsäure oder einer Kombination davon,
wobei eine minimale Subsiedetemperatur 160 °C weniger als der Normalsiedepunkt ist, falls der Normalsiedepunkt des organischen Amins mindestens 200 °C ist, wobei eine minimale Subsiedetemperatur 120 °C weniger als der Normalsiedepunkt ist, falls der Normalsiedepunkt des organischen Amins zwischen 150 °C und 200 °C liegt, und wobei eine minimale Subsiedetemperatur größer als 25 °C ist, falls der Normalsiedepunkt des organischen Amins weniger als 150 °C ist.

2. Verfahren nach Anspruch 1, wobei Schritte (c) und (d) vor Schritt (e) durchgeführt werden und wobei das organische Amin in Schritt (e) in der Flüssigkeit aus Schritt (d) ist.

3. Verfahren nach Anspruch 1, wobei Schritt (e) vor Schritten (a) - (d) durchgeführt wird und wobei das organische Amin dem ersten Gefäß in Schritt (a) nach dem Inberührungbringen mit der Harzpolymermatrix bereitgestellt wird.

4. Verfahren nach einem der vorstehenden Ansprüche, wobei, nachdem die Verfahrensschritte abgeschlossen sind, die Konzentration von Na, K, Ca, Al, Fe, Ni, Zn, Cu, Cr und Sn in dem organischen Amin jeweils 10 ppb oder weniger beträgt.

5. Verfahren nach einem der vorstehenden Ansprüche, wobei ein Wassergehalt und ein Sauerstoffgehalt in dem ersten Gefäß jeweils weniger als 20 ppm betragen.

6. Verfahren nach einem der vorstehenden Ansprüche, wobei die Harzpolymermatrix Polyacrylat oder Polystyroldivinylbenzol umfasst.

7. Verfahren nach einem der vorstehenden Ansprüche, wobei die Porengröße der Harzpolymermatrix in einem Bereich von 1 - 2.000 nm liegt, wie durch eine spezifische Oberfläche von Feststoffen durch Gasadsorption bestimmt.

8. Verfahren nach einem der vorstehenden Ansprüche, wobei die Harzpolymermatrix in die ein organisches Amin enthaltende Flüssigkeit als Harzperlen eingebracht wird, wobei eine Größe des Teilchendurchmessers der Perlen in dem Bereich von 100 - 2000 µm liegt.

9. Verfahren nach einem der vorstehenden Ansprüche, wobei das organische Amin hochkonzentriertes Monoethanolamin, Diethanolamin, Triethanolamin, Isopropanolamin, Diisopropanolamin, Triisopropanolamin, Dimethylethanolamin, N-Methyldiethanolamin oder Aminoethylethanolamin einschließt.

## Revendications

1. Procédé permettant de purifier des amines organiques, le procédé comprenant :
(a) la fourniture d'une amine organique à un premier récipient, l'amine organique ayant un point d'ébullition normal à un bar ;
(b) le remplissage du premier récipient avec un gaz inerte, dans lequel le gaz inerte est de l'azote ou de l'argon ;
(c) le chauffage de l'amine organique dans le premier récipient à une température inférieure à ébullition, dans lequel la température inférieure à ébullition est inférieure d'au moins 15 °C au point d'ébullition normal ;
(d) le refroidissement de la vapeur à partir du premier récipient dans un second récipient pour fournir un liquide ; et
(e) la mise en contact de l'amine organique avec une matrice polymère de résine, dans lequel la matrice polymère de résine est incorporée avec un composé aminé choisi dans le groupe constitué d'acide iminodiacétique, d'acide aminométhylphosphonique ou d'une combinaison de ceux-ci,
dans lequel une température inférieure à ébullition minimale est inférieure de 160 °C au point d'ébullition normal si le point d'ébullition normal de l'amine organique est d'au moins 200 °C, dans lequel une température inférieure à ébullition minimale est inférieure de 120 °C au point d'ébullition normal si le point d'ébullition normal de l'amine organique est compris entre 150 °C et 200 °C, et dans lequel une température inférieure à ébullition minimale est supérieure à 25 °C si le point d'ébullition normal de l'amine organique est inférieur à 150 °C.

2. Procédé selon la revendication 1, dans lequel les étapes (c) et (d) sont réalisées avant l'étape (e), et dans lequel l'amine organique de l'étape (e) est dans le liquide de l'étape (d).

3. Procédé selon la revendication 1, dans lequel l'étape (e) est réalisée avant les étapes (a) à (d), et dans lequel l'amine organique est fournie au premier récipient à l'étape (a) après avoir été mise en contact avec la matrice polymère de résine.

4. Procédé selon l'une quelconque des revendications précédentes, dans lequel, une fois les étapes de procédé terminées, les concentrations de Na, K, Ca, Al, Fe, Ni, Zn, Cu, Cr et Sn dans l'amine organique sont chacune égale ou inférieure à 10 ppb.

5. Procédé selon l'une quelconque des revendications précédentes, dans lequel une teneur en eau et une teneur en oxygène dans le premier récipient sont chacune inférieures à 20 ppm.

6. Procédé selon l'une quelconque des revendications précédentes, dans lequel la matrice polymère de résine comprend du polyacrylate ou du polystyrène-divinylbenzène.

7. Procédé selon l'une quelconque des revendications précédentes, dans lequel la taille des pores de la matrice polymère de résine est comprise entre 1 et 2 000 nm, telle que déterminée par la surface spécifique des matières solides par adsorption de gaz.

8. Procédé selon l'une quelconque des revendications précédentes, dans lequel la matrice polymère de résine est introduite dans le liquide contenant une amine organique sous forme de grains de résine, le diamètre de particule desdits grains allant de 100 à 2 000 µm.

9. Procédé selon l'une quelconque des revendications précédentes, dans lequel l'amine organique comporte de la monoéthanolamine, de la diéthanolamine, de la triéthanolamine, de l'isopropanolamine, de la diisopropanolamine, de la triisopropanolamine, de la diméthyléthanolamine, de la N-méthyldiéthanolamine ou de l'aminoéthylèneéthanolamine hautement concentrées.
